# FASCICULE DE BREVET EUROPEEN

(11) **EP 1 065 209 B1**
(45) Date de publication et mention de la délivrance du brevet: **02.05.2003**
(21) Numéro de dépôt: 00401864.4
(22) Date de dépôt: 30.06.2000
(51) Int. Cl.: C07D 491/048, A61K 31/4355, A61P 35/00

(54) **Nouveaux inhibiteurs de métalloprotéases, leur procédé de préparation et les compositions pharmaceutiques qui les contiennent**
Metalloproteaseinhibitoren, Verfahren zu ihrer Herstellung und diese enthaltende pharmazeutische Zubereitungen
Metalloprotease inhibitors, process for their preparation and pharmaceutical compositions containing them

(30) Priorité: 01.07.1999 FR 9908448
(43) Date de publication de la demande: 03.01.2001
(73) Titulaire: LES LABORATOIRES SERVIER, 92200 Neuilly sur Seine (FR)
(72) Inventeur: de Nanteuil, Guillaume, 92150 Suresnes (FR); Benoist, Alain, 95130 Franconville (FR); Bonnet, Jacqueline, 75013 Paris (FR); Sabatini, Massimo, 92380 Garches (FR); Atassi, Ghanem, 92210 Saint Cloud (FR); Pierre, Alain, 78580 Les Alluets Le Roi (FR)

(56) Documents cités:
- EP-A- 0 803 505
- WO-A-99/06410

## Description

La présente invention concerne de nouveaux inhibiteurs de métalloprotéases, leur procédé de préparation et les compositions pharmaceutiques qui les contiennent.

A l'état physiologique, la synthèse des tissus connectifs est en équilibre dynamique avec la dégradation de la matrice extracellulaire. Cette dégradation est due à des protéases à zinc (métalloprotéases) secrétées par les cellules de la matrice existante : ce sont de façon non limitative les collagénases (MMP-1, MMP-8, MMP-13), les gélatinases ou collagénases de type IV (MMP-2, MMP-9) et les stromélysines (MMP-3).

A l'état normal, ces enzymes cataboliques sont régulées au niveau de leur synthèse et de leur sécrétion, ainsi qu'au niveau de leur activité enzymatique extracellulaire par des inhibiteurs naturels, comme l'α₂-macroglobuline ou les TIMP (Tissue Inhibitor of MetalloProteinase) qui forment des complexes inactifs avec les métalloprotéases.

Le point commun aux pathologies impliquant ces enzymes est un déséquilibre entre l'activité des enzymes activées et celle de leurs inhibiteurs naturels, avec pour conséquence une dégradation excessive des tissus.

La dégradation incontrôlée et accélérée des membranes par la résorption de la matrice extracellulaire catalysée par les métalloprotéases est un paramètre commun à plusieurs conditions pathologiques comme l'arthrite rhumatoïde, l'arthrose, l'invasion et la croissance tumorale, y compris la dissémination maligne et la formation de métastases, les ulcérations, l'athérosclérose, etc...

Récemment, le BB94, inhibiteur de métalloprotéases a montré une activité antitumorale en clinique où il s'est révélé actif sur les cancers de l'ovaire (Becket et al., DDT 1996, 1 (1), 16).

On peut donc attendre d'un inhibiteur de métalloprotéases qu'il restaure l'équilibre entre protéase et inhibiteur et de ce fait modifie de façon favorable l'évolution de ces pathologies.

Un certain nombre d'inhibiteurs de métalloprotéases ont été décrits dans la littérature. C'est le cas, plus particulièrement, des composés décrits dans les brevets WO 95/35275, WO 95/35276, EP 606 046, WO 96/00214 ou EP 803 505.

Les composés de la présente invention, outre le fait qu'ils soient nouveaux, se sont révélés être des inhibiteurs de métalloprotéases plus puissants que ceux décrits dans la littérature ce qui les rend donc potentiellement utiles pour le traitement des cancers, des maladies rhumatismales comme l'arthrose et l'arthrite rhumatoïde, de l'athérosclérose, etc...

Plus spécifiquement, la présente invention concerne les composés de formule (I) : 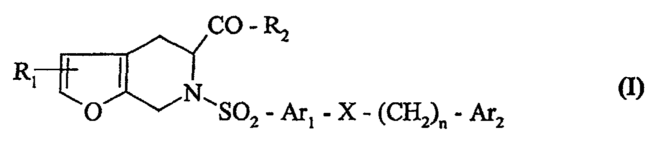 dans laquelle :
- R₁: représente un atome d'hydrogène, d'halogène, un groupement alkyle (C₁-C₆) linéaire ou ramifié, ou alkoxy (C₁-C₆) linéaire ou ramifié,
- R₂: représente un groupement hydroxy, alkoxy (C₁-C₆) linéaire ou ramifié ou -NHOH,
- Ar₁: représente un groupement phénylène ou biphénylène,
- X: représente un atome d'oxygène, ou de soufre,
- R: représente un atome d'hydrogène ou un groupement alkyle (C₁-C₆) linéaire ou ramifié,
- n: représente un nombre entier compris entre 0 et 6 inclus,
- Ar₂: représente :
- un groupement phényle substitué par un groupement hétéroaryle,
- un groupement biphényle substitué par un groupement hétéroaryle,
- un groupement pyridinyle, substitué par un groupement hétéroaryle,
- ou un groupement hétérocyclique,
leurs isomères ainsi que leurs sels d'addition à un acide ou à une base pharmaceutiquement acceptable,
étant entendu que :
- par groupement hétéroaryle, on comprend groupement aromatique monocyclique ou un groupement bicyclique dans lequel au moins un des cycles est aromatique, contenant un, deux ou trois hétéroatomes, identiques ou différents, choisis parmi azote, oxygène ou soufre, éventuellement substitué par un ou plusieurs groupements, identiques ou différents, choisis parmi halogène, alkyle (C₁-C₆) linéaire ou ramifié, alkoxy (C₁-C₆) linéaire ou ramifié, trihalogénoalkyle (C₁-C₆) linéaire ou ramifié, trihalogénoalkoxy (C₁-C₆) linéaire ou ramifié, ou hydroxy,
- par groupement hétérocyclique, on comprend un groupement saturé ou partiellement saturé, non aromatique mono ou bicyclique contenant un, deux ou trois hétéroatomes, identiques ou différents, choisis parmi azote, oxygène ou soufre, éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, alkoxy (C₁-C₆) linéaire ou ramifié, trihalogénoalkoxy (C₁-C₆) linéaire ou ramifié, ou hydroxy.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les groupements hétéroaryles préférés sont les groupements imidazolyle, thiazolyle, oxazolyle, pyrrolyle, pyridyle, pyrimidyle, triazolyle, pyrazolyle, benzimidazolyle.

Les groupements hétérocycliques préférés sont les groupements pyrrolidinyle, morpholino, pipéridino, imidazolidinyle, thiazolidinyle, oxazolidinyle, pipérazinyle, isoindolyle, 2,3-dihydroisoindolyle, cyclopenta[c]pyrrolidinyle.

Le groupement R₁ préféré est l'atome d'hydrogène.
Le groupement R₂ préféré est le groupement -NHOH.

Lorsque Ar₁ représente un groupement phénylène, n représente plus particulièrement zéro.

Lorsque Ar₁ représente un groupement phénylène, Ar₂ représente de manière préférentielle un groupement phényle substitué par un groupement hétéroaryle, le groupement hétéroaryle étant préférentiellement un groupement imidazolyle, triazolyle ou pyridinyle.

Plus particulièrement, les composés préférés de l'invention sont les composés de formule (I) dans laquelle Ar₁ représente un groupement phénylène, X représente un atome d'oxygène ou de soufre, n représente zéro, Ar₂ représente un groupement phényle substitué par un groupement hétéroaryle choisi parmi imidazolyle, triazolyle ou pyridinyle.

Lorsque Ar₁ représente un groupement biphénylène, Ar₂ représente de manière préférentielle un groupement hétérocyclique.

La configuration du cycle 4,5,6,7-tétrahydrofuro[2,3-*c*]pyridine est préférentiellement (5R).

Les composés préférés de l'invention sont :
- le 6-{4-[4-(imidazol-1-yl)phénoxy]benzènesulfonyl}-4,5,6,7-tétrahydrofuro[2,3-*c*] pyridine-(5R)-(N-hydroxy)carboxamide, ainsi que ses sels d'additions correspondants,
- le 6-{4'-[2-(pyrrolidin-1-yl)éthoxy]biphényl-4-sulfonyl}-4,5,6,7-tétrahydrofuro[2,3-c] pyridine-(5R)-(N-hydroxy)carboxamide, ainsi que ses sels d'addition correspondants,
- le 6-{4-[4-(1,3,4-triazol-1-yl)phénoxy]benzènesulfonyl}-4,5,6,7-tétrahydrofuro[2,3-c] pyridin-(5R)-(N-hydroxy)carboxamide, ainsi que ses sels d'addition correspondants,
- le 6-{4-[4-(pyridin-4-yl)phénoxy]benzènesulfonyl}-4,5,6,7-tétrahydrofuro[2,3-c]pyridin-(5R)-(N-hydroxy)carboxamide, ainsi que ses sels d'addition correspondants,
- le 6-{4-[(4-(1,3,4-triazol-1-yl)phénythio]benzènesulfonyl}-4,5,6,7-tétrahydrofuro[2,3-c] pyridine-(5R)-(N-hydroxy)carboxamide, ainsi que ses sels d'addition correspondants.

L'invention s'étend également au procédé de préparation des composés de formule (I) caractérisé en ce que l'on utilise comme produit de départ un composé de formule (II), sous forme racémique ou d'isomère défini : 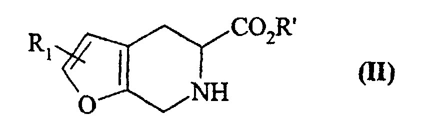 dans laquelle R₁ a la même signification que dans la formule (I), et R' représente un atome d'hydrogène ou un groupement alkyle (C₁-C₆) linéaire ou ramifié, dont on substitue la fonction amine par un dérivé halogéné de formule (III) :

ClSO₂-AR₁-X-(CH₂)ₙ-Ar₂ **(III)**

dans laquelle Ar₁, X, n et Ar₂ ont la même signification que dans la formule (I), pour conduire :
- lorsque R' représente un atome d'hydrogène au composé de formule (I/a), cas particulier des composés de formule (I) : 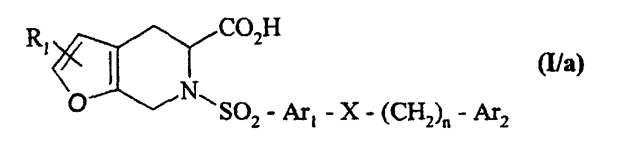 dans laquelle R₁, Ar₁, X, N et Ar₂ sont tels que définis précédemment,
- ou, lorsque R' représente un groupement alkyle (C₁-C₆) linéaire ou ramifié (R") au composé de formule (I/a₁), cas particulier des composés de formule (I) : 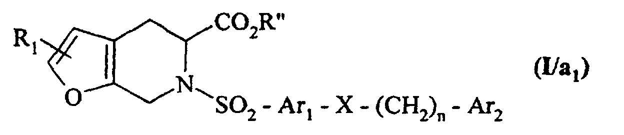 dans laquelle R₁, Ar₁, X, n, Ar₂ et R" sont tels que définis précédemment, qui peut subir l'action d'un acide, pour conduire au composé de formule (I/a) décrit précédemment,
   * composé de formule (I/a) :
      que l'on soumet, si on le souhaite, alors à l'action d'une hydroxylamine -O- substituée, pour conduire, après déprotection de la fonction hydroxylamine au composé de formule (I/b), 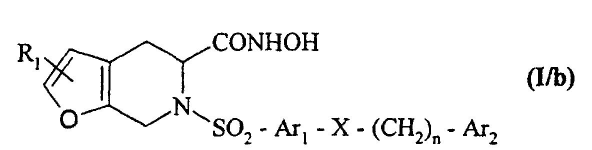 dans laquelle R₁, Ar₁, X, n et Ar₂ sont tels que définis précédemment,
   * composés de formule (I/a), (I/a₁) ou (I/b) qui forment l'ensemble des composés de formule (I),
      que l'on purifie le cas échéant selon une technique classique de purification, dont on sépare éventuellement les isomères selon une technique classique de séparation et dont on transforme, si on le souhaite, en ses sels d'addition à un acide ou à une base pharmaceutiquement acceptable.
Les composés de formule (II) et (III) sont, soit des composés commerciaux, soit obtenus selon des modes opératoires connus.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 0,01 à 2 g par jour en une ou plusieurs prises.

Les exemples suivants illustrent l'invention mais ne la limitent en aucune façon.

Les produits de départ utilisés sont des produits connus ou préparés selon des modes opératoires connus.

Les préparations conduisent à des intermédiaires de synthèse, utiles pour la préparation des composés de l'invention.

Les structures des composés décrits dans les exemples et les préparations ont été déterminées selon les techniques spectrophotométriques usuelles (infrarouge, RMN, spectrométrie de masse, ...).

### PREPARATION A : Chlorure de l'acide 4-[4-(Imidazol-1-yl)phénoxy]benzène sulfonique

### Stade A : Acide 4-[4-(imidazol-1-yl)phénoxy]benzènesulfonique

137 mmoles de 1-(4-phénoxyphényl)imidazole sont dissoutes dans 250 ml de chloroforme. 190 mmoles d'acide chlorosulfonique sont alors additionnées goutte à goutte. L'ensemble est laissé à 45°C pendant une nuit. Après refroidissement et évaporation du solvant, l'huile obtenue est reprise par de l'éther éthylique. Le solide obtenu est filtré et séché et conduit au produit attendu.
***Point de fusion : 80°C***

### Stade B : Chlorure de l'acide 4-[4-(Imidazol-1-yl)phénoxy]benzènesulfonique

80 mmoles du produit décrit au stade précédent et 90 mmoles de PCl₅ sont placées dans 100 ml de POCl₃. La suspension est chauffée 3 heures à reflux. Après refroidissment, le résidu huileux est lavé à l'éther puis repris par de l'acétonitrile. Le précipité obtenu est filtré, lavé à l'éther isopropylique et conduit au produit du titre.
***Point de fusion : 170°C***

### PREPARATION B : Chlorure de l'acide 4'-[2-(pyrrolidin-1-yl)éthoxy]-biphényl-4-sulfonique

### Stade A : 1-[2-(Biphényloxy)éthyl]pyrrolidine

1,76 moles de 4-hydroxybiphényle, 2,29 moles de 1-(2-chloroéthyl)pyrrolidine et 5,3 moles de carbonate de potassium sont placées dans 2,5 l de diméthylformamide. L'ensemble est chauffé à 50°C une nuit. Après refroidissement, le solide est filtré et le solvant évaporé. Le résidu est repris par de l'acétate d'éthyle. Après lavage de la phase organique, séchage et évaporation, on obtient un résidu qui est purifié par chromatographie sur silice en utilisant comme éluant un mélange acétate d'éthyle/éthanol (80/20). Le produit attendu est obtenu après cristallisation de l'huile résiduelle.

### Stade B : Acide 4'-[2-(pyrrolidin-1-yl)éthoxy]biphényl-4-sulfonique

Le produit attendu est obtenu selon le procédé décrit au stade B de la préparation A à partir du produit décrit au stade A.

### Stade C : Chlorure de l'acide 4'-[2-(pyrrolidin-1-yl)éthoxy]-biphényl-4-sulfonique

Le produit attendu est obtenu selon le procédé décrit au stade C de la préparation A à partir du composé décrit au stade B en maintenant le reflux pendant 9 heures et filtration du précipité obtenu.
***Point de fusion : 234°C***

### PREPARATION C : Chlorure de l'acide 4'-[(pyridin-4-yl)phénoxy]biphényl-4-sulfonqiue

Le produit attendu est obtenu selon le procédé décrit dans la préparation B en utilisant au stade A la 4-(4-chlorophényl)pyridine à la place de la 1-(2-chloroéthyl)pyrrolidine.

### PREPARATION D : Chlorure de l'acide 4'-[(2-morpholino)éthoxy]biphényl-4-sulfonique

Le produit attendu est obtenu selon le procédé décrit dans la préparation B en utilisant au stade A la 4-(2-chloroéthyl)morpholine à la place de la 1-(2-chloroéthyl)pyrrolidine.

### PREPARATION E : Chlorure de l'acide 4'-[(2-pipéridino)éthoxy]-biphényl-4-sulfonique

Le produit attendu est obtenu selon le procédé décrit dans la préparation B en utilisant au stade A la 1-(2-chloroéthyl)pipéridine à la place de la 1-(2-chloroéthyl)pyrrolidine.

### PREPARATION G : Chlorure de l'acide 4'-[2-(perhydroazépin-1-yl)éthoxy]biphényl-4 sulfonique

Le produit attendu est obtenu selon le procédé décrit dans la préparation B en utilisant au stade A la 1-(2-chloroéthyl)perhydroazépine à la place de la 1-(2-chloroéthyl)pyrrolidine.

### PREPARATION H : Chlorure de l'acide 4'-[3-(pyrrolidin-1-yl)propoxy]biphényl-4-sulfonique

Le produit attendu est obtenu selon le procédé décrit dans la préparation B en utilisant au stade A la 1-(3-chloropropyl)pyrrolidine à la place de la 1-(2-chloroéthyl)pyrrolidine.

### PREPARATION I : Chlorure de l'acide 4'-[2-(1,3-dihydroisoindol-2-yl)éthoxy]biphényl-4-sulfonique

Le produit attendu est obtenu selon le procédé décrit dans la préparation B en utilisant au stade A le 2-(2-chloroéthyl)-1,3-dihydroisoindole à la place de la 1-(2-chloroéthyl)pyrrolidine.

### PREPARATION J : Chlorure de l'acide 4'-[2-(cyclopenta[c]pyrrolidin-2-yl)) éthoxy]biphényl-4-sulfonique

Le produit attendu est obtenu selon le procédé décrit dans la préparation B en utilisant au stade A le 2-(2-chloroéthyl)-cyclopenta[c]pyrrolidine à la place de la 1-(2-chloroéthyl)pyrrolidine.

### PREPARATION O : Chlorure de l'acide 4'-[2-(perhydroazépin-1-yl)éthoxy]biphényl-4-sulfonique

Le produit attendu est obtenu selon le procédé décrit dans la préparation B en utilisant au stade A le 1,3-dihydroisoindole à la place de la 1-(2-chloroéthyl)pyrrolidine et le 4-bromobiphényle à la place du 4-hydroxybiphényle.

### PREPARATION P : Chlorure de l'acide 4-[4-(1,3,4-triazol-1-yl)phénoxy]benzènesulfonique

Le produit attendu est obtenu selon le procédé décrit dans la préparation A en utilisant au stade A le l-(4-phénoxyphényl)-1,3,4-triazole à la place du 1-(4-phénoxyphényl) imidazole.

### PREPARATION Q : Chlorure de l'acide 4-[4-(1,2,4-triazol-1-yl)phénoxy] benzènesulfonique

Le produit attendu est obtenu selon le procédé décrit dans la préparation A en utilisant au stade A le 1-(4-phénoxyphényl)-1,2,4-triazole à la place du 1-(4-phénoxyphényl) imidazole.

### PREPARATION R : Chlorure de l'acide 4-[4-(pyrrol-1-yl)phénoxy]benzènesulfonique

Le produit attendu est obtenu selon le procédé décrit dans la préparation A en utilisant au stade A le 1-(4-phénoxyphényl)pyrrole à la place du 1-(4-phénoxyphényl) imidazole.

### PREPARATION S : Chlorure de l'acide 4-[4-(pyrazol-1-yl)phénoxy]benzènesulfonique

Le produit attendu est obtenu selon le procédé décrit dans la préparation A en utilisant au stade A le 1-(4-phénoxyphényl)pyrazole à la place du 1-(4-phénoxyphényl) imidazole.

### PREPARATION T : Chlorure de l'acide 4-[4-(imidazol-2-yl)phénoxy]benzènesulfonique

Le produit attendu est obtenu selon le procédé décrit dans la préparation A en utilisant au stade A le 2-(4-phénoxyphényl)imidazole à la place du 1-(4-phénoxyphényl) imidazole.

### PREPARATION U : Chlorure de l'acide 4-[4-(benzimidazol-1-yl)phénoxy] benzènesulfonique

Le produit attendu est obtenu selon le procédé décrit dans la préparation A en utilisant au stade A le 1-(4-phénoxyphényl)benzimidazole à la place du 1-(4-phénoxyphényl) imidazole.

### PREPARATION V : Chlorure de l'acide 4-[4-(pyridin-4-yl)phénoxy]benzènesulfonique

Le produit attendu est obtenu selon le procédé décrit dans la préparation A en utilisant au stade A la 4-(4-phénoxyphényl)pyridine à la place du 1-(4-phénoxyphényl) imidazole.

### PREPARATION W : Chlorure de l'acide 4-[4-(pyrimidin-5-yl)phénoxy] benzènesulfonique

Le produit attendu est obtenu selon le procédé décrit dans la préparation A en utilisant au stade A la 5-(4-phénoxyphényl)pyrimidine à la place du 1-(4-phénoxyphényl) imidazole.

### PREPARATION X : Chlorure de l'acide 4-[4-(pyrimidin-2-yl)phénoxy] benzènesulfonique

Le produit attendu est obtenu selon le procédé décrit dans la préparation A en utilisant au stade A la 2-(4-phénoxyphényl)pyrimidine à la place du 1-(4-phénoxyphényl) imidazole.

### PREPARATION Y : Chlorure de l'acide 4-[2-(imidazol-1-yl)pyridin-5-yloxy] benzènesulfonique

Le produit attendu est obtenu selon le procédé décrit dans la préparation A en utilisant au stade A le 1-(5-phénoxypyridin-2-yl)imidazole à la place du 1-(4-phénoxyphényl) imidazole.

### PREPARATION Z : Chlorure de l'acide 4-[5-(imidazol-1-yl)pyridin-2-yloxy] benzènesulfonique

Le produit attendu est obtenu selon le procédé décrit dans la préparation A en utilisant au stade A le 1-(2-phénoxypyridin-5-yl)imidazole à la place du 1-(4-phénoxyphényl) imidazole.

### PREPARATION AA : Chlorure de l'acide 4-[4'-(imidazol-1-yl)biphényl-4-oxy] benzènesulfonique

Le produit attendu est obtenu selon le procédé décrit dans la préparation A en utilisant au stade A le 1-[4'-phénoxy-4-biphényl]imidazole à la place du 1-(4-phénoxyphényl) imidazole.

### PREPARATION AB : Chlorure de l'acide 4'-[4-(imidazol-1-yl)phénoxy]biphényl-4-sulfonique

Le produit attendu est obtenu selon le procédé décrit dans la préparation B en utilisant au stade A le 1-(4-chlorophényl)imidazole à la place de la 1-(2-chloroéthyl)pyrrolidine.

### PREPARATION AC : Chlorure de l'acide 4-[(4-chloropyrazol-1-yl)phénoxy]benzène sulfonique

Le produit attendu est obtenu selon le procédé décrit dans la préparation A en utilisant au stade A le 1-(4-phénoxyphényl)-4-chloropyrazole à la place du 1-(4-phénoxyphényl) imidazole.

### PREPARATION AD : Chlorure de l'acide 4-[4-(imidazol-1-yl)phénylthio]benzène sulfonique

Le produit attendu est obtenu selon le procédé décrit dans la préparation A en utilisant au stade A le 1-(4-phénylthiophényl)imidazole à la place du 1-(4-phénoxyphényl)imidazole.

### PREPARATION AE : Chlorure de l'acide 4-[4-(pyridin-3-yl)phénoxy]benzène sulfonique

Le produit attendu est obtenu selon le procédé décrit dans la préparation A en utilisant au stade A la 3-(4-phénoxyphényl)pyridine à la place du 1-(4-phénoxyphényl)imidazole.

### PREPARATION AF : Chlorure de l'acide 4-[4-(1,3,4-triazol-1-yl)phénylthio]benzène sulfonique

Le produit attendu est obtenu selon le procédé décrit dans la préparation A en utilisant au stade A le 1-(4-phénylthiophényl)-1,3,4-triazole à la place du 1-(4-phénoxyphényl) imidazole.

### EXEMPLE 1 : Acide 6-{4-[4-(imidazol-1-yl)phénoxy]benzènesulfonyl}-4,5,6,7-tétrahydrofuro[2,3-c]pyridine-(5R)-carboxylique, sel de sodium

### Stade A : tert-Butyl ester de l'acide 6-{4-[4-(imidazol-1-yl)phénoxy]benzènesulfonyl}-4,5,6,7-tétrahydrofuro[2,3-c]pyridine-(5R)carboxylique

30 mmoles de *tert*-butyl ester de l'acide 4,5,6,7-tétrahydrofuro[2,3-c]pyridine-(5R)carboxylique (obtenu selon la méthode décrite par M. S. Allen, Synth. Comm., 22 (14), 2077-2102, 1992) sont dissoutes dans 60 ml de pyridine. 33 mmoles du composé décrit dans la préparation A sont alors ajoutées par fractions. Le mélange est agité à température ambiante une nuit, puis versé dans 300 ml d'eau.
Après extraction à l'acétate d'éthyle, la phase organique est lavée l'eau puis séchée. Après filtration et évaporation du solvant, le résidu est purifié par chromatographie sur gel de silice en utilisant comme éluant un mélange acétate d'éthyle/pentane (8/2) et conduit au produit attendu.

### Stade B : Acide 6-{4-[4-(imidazol-1-yl)phénoxy]benzènesulfonyl}-4,5,6,7-tétrahydrofuro[2,3-c]pyridine-(5R)-carboxylique, sel de sodium

10 mmoles d'ester obtenues au stade A sont dissoutes dans 70 ml de chlorure de méthylène. 10 mmoles d'anisole sont alors ajoutées, puis à 0°C, 70 ml d'acide trifluoroacétique. La réaction est ensuite agitée à température ambiante une nuit. Après évaporation, le résidu obtenu est purifié par chromatographie sur gel de silice en utilisant comme éluant un mélange dichlorométhane/éthanol (9/1) et conduit au produit attendu après lyophilisation du sel de sodium correspondant.

| *Microanalyse élémentaire :* | | | | |
|---|---|---|---|---|
| | *C %* | *H %* | *N %* | *S %* |
| *calculé* | *56,67* | *3,72* | *8,62* | *6,58* |
| *trouvé* | *56,93* | *3,81* | *8,59* | *6,86* |

### EXEMPLE 2 : 6-{4-[4-(Imidazol-1-yl)phénoxy]benzènesulfonyl}-4,5,6,7-tétrahydrofuro[2,3-c]pyridine-(5R)-(N-hydroxy)carboxamide, chlorhydrate

### Stade A : 6-{4-[4-(Imidazolyl-1-yl)phénoxy]benzènesulfonyl}-4,5,6,7-tétrahydrofuro [2,3-c]pyridine-(5R)-(N-allyloxy)carboxamide

9 mmoles du composé décrit dans l'exemple 1 sont dissoutes dans 70 ml de dichlorométhane et 10 ml de diméthylformamide. On ajoute alors au mélange précédent 45 mmoles de diisopropyléthylamine, 9 mmoles de dihydroxybenzotriazole, 13 mmoles de chlorhydrate O-allylhydroxylamine et 11 mmoles de O-(benzotriazol-1-yl)-N,N,N',N'-tétraméthyl-uronium tétrafluoborate.
Après une nuit à température ambiante, le mélange est évaporé. Le résidu est repris par du chlorométhane. Après séchage, la solution est filtrée et évaporée. Le résidu est purifié par chromatographie sur gel de silice en utilisant comme éluant un mélange dichlorométhane/éthanol (95/5) et conduit au produit attendu.

### Stade B : 6-{4-[4-(Imidazol-1-yl)phénoxy]benzènesulfonyl}-4,5,6,7-tétrahydrofuro[2,3-c]pyridine-(5R)-(N-hydroxy)carboxamide, chlorhydrate

8,5 mmoles du composé décrit au stade précédent sont dissoutes dans 120 ml de dichlorométhane. 0,4 mmoles de (Ph₃P)₂PdCl₂ et 2,5 mmoles d'acide acétique sont ajoutées au mélange précédent et l'ensemble est agité 5 minutes avant l'addition de 4,8 ml d'hydrure de tributylétain. Après 5 minutes d'agitation, le solvant est évaporé et le résidu est repris par un mélange acétonitrile/méthanol. Après lavage à l'hexane et évaporation, le résidu est purifié sur colonne chromatographique phase inverse en utilisant comme éluant un mélange acétonitrile/méthanol. Après lyophilisation, le solide obtenu est dissous dans de l'acétonitrile et est transformé en chlorhydrate correspondant par addition de 4,32 ml d'HCl 1N. Le produit du titre est alors obtenu après lyophilisation.

| *Microanalyse élémentaire :* | | | | | |
|---|---|---|---|---|---|
| | *C %* | *H %* | *N %* | *Cl %* | *S %* |
| *calculé* | *53,44* | *4,09* | *10,84* | *6,86* | *6,20* |
| *trouvé* | *54,05* | *4,13* | *10,82* | *6,61* | *6,07* |

Les exemples suivants ont été préparés selon les procédés décrits dans l'exemple 1 ou 2 à partir des produits de départ correspondants.

### EXEMPLE 3 : Acide 6-{4'-[2-(Pyrrolidin-1-yl)éthoxy]biphényl-4-sulfonyl}-4,5,6,7-tétrahydrofuro[2,3-c}]pyridine-(5R)-carboxylique, sel de sodium

Le produit du titre est obtenu selon le procédé décrit dans l'exemple 1 à partir du produit décrit dans la préparation B.

| *Microanalyse élémentaire :* | | | | |
|---|---|---|---|---|
| | *C %* | *H %* | *N %* | *S %* |
| *calculé* | *55,08* | *4,79* | *4,59* | *5,25* |
| *trouvé* | *54,83* | *4,50* | *4,57* | *5,09* |

### EXEMPLE 4 : 6-{4'-[2-(Pyrrolidin-1-yl)éthoxy]biphényl-4-sulfonyl}-4,5,6,7-tétrahydrofuro[2,3-c]pyridine- (5R )-(N-hydroxy)carboxamide, chlorhydrate

Le produit du titre est obtenu selon le procédé décrit dans l'exemple 2 à partir du produit décrit dans l'exemple 3.

| *Microanalyse élémentaire :* | | | | | |
|---|---|---|---|---|---|
| | *C %* | *H %* | *N %* | *Cl %* | *S %* |
| *calculé* | *56,98* | *5,52* | *7,67* | *6,47* | *5,85* |
| *trouvé* | *56,64* | *5,49* | *7,48* | *7,14* | *5,59* |

### EXEMPLE 5 : Acide 6-{4'[4-(pyridin-4-yl)phénoxy]biphényl-4-sulfonyl}-4,5,6,7-tétrahydrofuro[2,3-c]pyridine-(5R)carboxylique, sel de sodium

Le produit du titre est obtenu selon le procédé décrit dans l'exemple 1 à partir du produit décrit dans la préparation C.

### EXEMPLE 6 : 6-{4'-[4-(Pyridin-4-yl)phénoxy]biphényl-4-sulfonyl}-4,5,6,7-tétrahydrofuro[2,3-c]pyridine-(5R)-(N-hydroxy)carboxamide, chlorhydrate

Le produit du titre est obtenu selon le procédé décrit dans l'exemple 2 à partir du produit décrit dans l'exemple 5.

### EXEMPLE 7 : Acide 6-{4'-[2-(morpholino)éthoxy]biphényl-4-sulfonyl}-4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation D.

### EXEMPLE 8 : 6-{4'-[2-(Morpholino)éthoxy]biphényl-4-sulfonyl}-4,5,6,7-tétrahydrofuro[2,3-c]pyridine-(5R)-(N-hydroxy)carboxamide, chlorhydrate

Le produit du titre est obtenu selon le procédé décrit dans l'exemple 2 à partir du produit décrit dans l'exemple 7.

| *Microanalyse élémentaire :* | | | | | |
|---|---|---|---|---|---|
| | *C%* | *H%* | *N%* | *Cl%* | *S%* |
| *calculé* | *55,36* | *5,36* | *7,45* | *6,29* | *5,68* |
| *trouvé* | *55,63* | *5,44* | *7,41* | *6,63* | *5,65* |

### EXEMPLE 9 : Acide 6-[4'-[2-(pipéridino)éthoxy]biphényl-4-sulfonyl}-4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation E.

### EXEMPLE 10 : 6-{4'-[2-(Pipéridino)éthoxy]biphényl-4-sulfonyl}-4,5,6,7-tétrahydrofuro[2,3-c]pyridine-(5R)-(N-hydroxy)carboxamide, chlorhydrate

Le produit du titre est obtenu selon le procédé décrit dans l'exemple 2 à partir du produit décrit dans l'exemple 9.

| *Microanalyse élémentaire :* | | | | | |
|---|---|---|---|---|---|
| | *C%* | *H%* | *N%* | *Cl%* | *S%* |
| *calculé* | *57,70* | *5,74* | *7,48* | *6,31* | *5,70* |
| *trouvé* | *57,97* | *5,79* | *7,47* | *6,54* | *5,55* |

### EXEMPLE 13 : Acide 6-{4'-[2-(perhydroazépin-1-yl)éthoxy]biphényl-4-sulfonyl} -4,5,6,7-tétrahydrofuro[2,3-c]pyridine-(5R) -carboxylique, sel de sodium

Le produit du titre est obtenu selon le procédé décrit dans l'exemple 1 à partir du produit décrit dans la préparation G.

### EXEMPLE 14 : 6-{4'-[2-(Perhydroazépin-1-yl)éthoxy]biphényl-4-sulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 13.

### EXEMPLE 15 : Acide 6-{4'-[3-pyrrolidin-1-ylpropoxy]biphényl-4-sulfonyl} -4,5,6,7-tétrahydrofuro[2,3-c]pyridine- (5R)-carboxylique, sel de sodium

Le produit du titre est obtenu selon le procédé décrit dans l'exemple 1 à partir du produit décrit dans la préparation H.

### EXEMPLE 16 : 6-{4'-[3-Pyrrolidin-1-ylpropoxy]biphényl-4-sulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 15.

### EXEMPLE 17: Acide 6-{4'-[2-(1,3-dihydroisoindol-2-yl)éthoxy]biphényl-4-sulfonyl]} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation I.

### EXEMPLE 18 : 6-{4'-[2-(1,3-Dihydroisoindol-2-yl)éthoxy]biphényl-4-sulfonyl]-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 17.

### EXEMPLE 19 : Acide 6-{4'-[2-(cyclopenta[c]pyrrolidin-2-yl)éthoxy]biphényl-4-sulfonyl}-4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation J.

### EXEMPLE 20 : 6-{4'-[2-(Cyclopenta[c]pyrrolidin-2-yl)éthoxy]biphényl-4-sulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 19.

### EXEMPLE 31 : Acide 6-{4-[4-(1,3,4-triazol-1-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation P.

### EXEMPLE 32 : 6-{4-[4-(1,3,4-Triazol-1-yl)phénoxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 31.

| *Microanalyse élémentaire :* | | | | |
|---|---|---|---|---|
| | *C%* | *H%* | *N%* | *S%* |
| *calculé* | *54,88* | *3,98* | *14,55* | *6,66* |
| *trouvé* | *54,88* | *4,01* | *14,16* | *6,57* |

### EXEMPLE 33 : Acide 6-{4-[4-(1,2,4-triazol-1-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation Q.

### EXEMPLE 34 : 6-{4-[4-(1,2,4-Triazol-1-yl)phénoxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 33.

| *Microanalyse élémentaire :* | | | | |
|---|---|---|---|---|
| | *C%* | *H%* | *N%* | *S%* |
| *calculé* | *54,88* | *3,98* | *14,55* | *6,66* |
| *trouvé* | *54,77* | *4,14* | *13,70* | *6,55* |

### EXEMPLE 35 : Acide 6-{4-[4-(pyrrol-1-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation R.

### EXEMPLE 36 : 6-{4-[4-(Pyrrol-1-yl)phénoxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 35.

### EXEMPLE 37 : Acide 6-{4-[4-(pyrazol-1-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation S.

### EXEMPLE 38 : 6-{4-[4-(Pyrazol-1-yl)phénoxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 37.

### EXEMPLE 39 : Acide 6-{4-[4-(imidazol-2-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation T.

### EXEMPLE 40 : 6-{4-[4-(Imidazol-2-yl)phénoxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 39.

### EXEMPLE 41 : Acide 6-{4-[4-(benzimidazol-1-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation U.

### EXEMPLE 42 : 6-{4-[4-(Benzimidazol-1-yl)phénoxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 41.

| *Microanalyse élémentaire :* | | | | | |
|---|---|---|---|---|---|
| | *C%* | *H%* | *N%* | *Cl%* | *S%* |
| *calculé* | *57,19* | *4,09* | *9,88* | *6,25* | *5,65* |
| *trouvé* | *57,31* | *4,10* | *9,86* | *5,61* | *5,66* |

### EXEMPLE 43 : Acide 6-{4-[4-(pyridin-4-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation V.

### EXEMPLE 44 : 6-{4-[4-(Pyridin-4-yl)phénoxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 43.

### EXEMPLE 45 : Acide 6-{4-[4-(pyrimidin-5-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation W.

### EXEMPLE 46 : 6-{4-[4-(Pyrimidin-5-yl)phénoxy]benzènesulfonyl}-4,5,6,7-tétrahydrofuro[2,3-c]pyridin- (5R)-(N-hydroxy)carboxamide, chlorhydrate

Le produit du titre est obtenu selon le procédé décrit dans l'exemple 2 à partir du produit décrit dans l'exemple 45.

| *Microanalyse élémentaire :* | | | | | |
|---|---|---|---|---|---|
| | *C%* | *H%* | *N%* | *S%* | *Cl%* |
| *calculé* | *54,50* | *4,00* | *10,59* | *6,06* | *6,70* |
| *trouvé* | *54,90* | *4,13* | *10,28* | *5,74* | *6,73* |

### EXEMPLE 47 : Acide 6-{4-[4-(pyrimidin-2-yl)phénoxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation X.

### EXEMPLE 48 : 6-{4-[4-(Pyrimidin-2-yl)phénoxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 47.

### EXEMPLE 49 : Acide 6-{4-[2-(imidazol-1-yl)pyridin-5-yloxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation Y.

### EXEMPLE 50 : 6-{4-[2-(Imidazol-1-yl)pyridin-5-yloxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 49.

### EXEMPLE 51 : Acide 6-{4-[5-(imidazol-1-yl)pyridin-2-yloxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation Z.

### EXEMPLE 52 : 6-{4-[5-(Imidazol-1-yl)pyridin-2-yloxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 51.

### EXEMPLE 53 : Acide 6-{4-[4'-(imidazol-1-yl)biphényl-4-oxy]benzènesulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation AA.

### EXEMPLE 54 : 6-{4-[4'-(Imidazol-1-yl)biphényl-4-oxy]benzènesulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 53.

| *Microanalyse élémentaire :* | | | | | |
|---|---|---|---|---|---|
| | *C%* | *H%* | *N%* | *S%* | *cl%* |
| *calculé* | *58,73* | *4,25* | *9,45* | *5,41* | *5,98* |
| *trouvé* | *59,09* | *4,27* | *9,23* | *5,21* | *5,97* |

### EXEMPLE 55 : Acide 6-{4'-[4-(imidazol-1-yl)phénoxy]biphényl-4-sulfonyl} -4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation AB.

### EXEMPLE 56 : 6-{4'-[4-(Imidazol-1-yl)phénoxy]biphényl-4-sulfonyl}-4,5,6,7-tétrahydrofuro[2,3-c]pyridin-(5R)-(N-hydroxy)carboxamide, chlorhydrate

Le produit du titre est obtenu selon le procédé décrit dans l'exemple 2 à partir du produit décrit dans l'exemple 55.

### EXEMPLE 57 : Acide 6-{4-[4-(4-chloropyrazol-1-yl)phénoxy]benzènesulfonyl}-4,5,6,7-tétrahydrofuro[2,3-c]pyridin-(5R)-carboxylique, sel de sodium

Le produit du titre est obtenu selon le procédé décrit dans l'exemple 1 à partir du produit décrit dans la préparation AC.

### EXEMPLE 58 : 6-{4-[4-Chloropyrazol-1-yl)phénoxy]benzènesulfonyl}-4,5,6,7-tétrahydrofuro[2,3-c]pyridin-(5R)-(N-hydroxy)carboxamide

Le produit du titre est obtenu selon le procédé décrit dans l'exemple 2 à partir du produit décrit dans l'exemple 57.

| *Microanalyse élémentaire :* | | | | | |
|---|---|---|---|---|---|
| | *C%* | *H%* | *N%* | *Cl%* | *S%* |
| *calculé* | *53,65* | *3,72* | *10,88* | *6,88* | *6,23* |
| *trouvé* | *53,40* | *3,81* | *10,64* | *6,88* | *6,07* |

### EXEMPLE 59 : Acide 6-{4-[4-(imidazol-1-yl)phénylthio]benzènesulfonyl}-4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation AD

### EXEMPLE 60 : 6-{4-(Imidazol-1-yl)phénylthio]benzènesulfonyl}-4,5,6,7-tétrahydrofuro [2,3-c]pyridine-(5R)-(N-hydroxy)carboxamide, chlorhydrate

Le produit du titre est obtenu selon le procédé décrit dans l'exemple 2 à partir du produit décrit dans l'exemple 59.

| *Microanalyse élémentaire :* | | | | | |
|---|---|---|---|---|---|
| | *C%* | *H%* | *N%* | *S%* | *Cl%* |
| *calculé* | *51,83* | *3,97* | *10,51* | *12,03* | *6,65* |
| *trouvé* | *51,04* | *3,99* | *10,11* | *12,26* | *7,65* |

### EXEMPLE 61 : Acide 6-{4-(pyridin-3-yl)phénoxy)benzènesulfonyl}-4,5,6,7-tétrahydrofuro[2,3-c]pyridine-(5R)carboxylique, sel de sodium

Le produit du titre est obtenu selon le procédé décrit dans l'exemple 1 à partir du produit décrit dans la préparation AE.

### EXEMPLE 62 : 6-{4-(Pyridin-3-yl)phénoxy)benzènesulfonyl}-4,5,6,7-tétrahydrofuro [2,3-c]pyridine-(5R)-(N-hydroxy)carboxamide, chlorhydrate

Le produit du titre est obtenu selon le procédé décrit dans l'exemple 2 à partir du produit décrit dans l'exemple 61.

| *Microanalyse élémentaire :* | | | | | |
|---|---|---|---|---|---|
| | *C%* | *H%* | *N%* | *S%* | *Cl%* |
| *calculé* | *56,87* | *4,20* | *7,96* | *6,07* | *6,71* |
| *trouvé* | *56,88* | *4,35* | *8,06* | *5,94* | *7,22* |

### EXEMPLE 63 : Acide 6-{4-[4-(1,3,4-triazol-1-yl)phénythio]benzènesulfonyl}-4,5,6,7-tétrahydrofuro[2,3-c]pyridine-(5R)-carboxylique, sel de sodium

Le produit du titre est obtenu selon le procédé décrit dans l'exemple 1 à partir du produit décrit dans la préparation AF.

### EXEMPLE 64 : 6-{4-[(4-(1,3,4-Triazol-1-yl)phénythio]benzènesulfonyl}-4,5,6,7-tétrahydrofuro[2,3-c]pyridine-(5R)-(N-hydroxy)carboxamide, chlorhydrate

Le produit du titre est obtenu selon le procédé décrit dans l'exemple 2 à partir du produit décrit dans l'exemple 63.

### EXEMPLE 65 : Ester éthylique de l'acide 6-{4-[4-(imidazol-1-yl) phénoxy]benzènesulfonyl}-4,5,6,7-tétrahydrofuro[2,3-c]pyridine-(5R)-carboxylique, chlorhydrate

Le produit attendu est obtenu selon le procédé décrit au stade A de l'exemple 1 à partir de l'ester éthylique de l'acide 4,5,6,7-tétrahydrofuro[2,3-c]pyridine-(5R)-carboxylique et du produit décrit dans la préparation A.

| *Microanalyse élémentaire :* | | | | | |
|---|---|---|---|---|---|
| | *C%* | *H%* | *N%* | *Cl%* | *S%* |
| *calculé* | *56,66* | *4,56* | *7,96* | *6,69* | *6,05* |
| *trouvé* | *56,80* | *4,67* | *7,94* | *6,88* | *5,87* |

### ETUDE PHARMACOLOGIQUE DES DERIVES DE L'INVENTION

### EXEMPLE A : Inhibition enzymatique des métalloprotéases

Les six enzymes humaines recombinantes -MMP-1 (collagénase interstitielle), MMP-2 (gélatinase A), MMP-3 (stromélysine 1), MMP-8 (collagénase des neutrophiles), MMP-9 (gélatinase B) et MMP-13 (collagénase 3)- sont activées à l'APMA (acétate 4-aminophénylmercurique).
Les tests enzymatiques des MMP-1, -2, -8, -9 et -13 sont effectués avec le substrat peptido mimétique suivant :

DnpProGhaGlyCys(Me)HisAlaLys(Nma)NH₂,

clivé entre la Glycine et la Cystéine pour produire un dérivé fluorescent décrit par D.M. BICKETT et coll. (Anal. Biochem., 212, 58-64, 1993).
Le test enzymatique de la MMP-3 est effectué avec le substrat peptido mimétique suivant :

McaArgProLysProTyrAlaNvaTrpMetLys(Dnp)NH₂,

clivé entre Alanine et Norvaline pour produite un dérivé fluorescent décrit par H. NAGASE et coll. (J. Biol. Chem., 269, 20952-20957, 1994).
Les réactions conduites dans un tampon 50 mM Tris, 200 mM NaCl, 5 mM CaCl₂, 0,1 % Brij 35 à pH 7.7 sont initiées avec 20 µM de substrat dans un volume total de 100 µl à 37°C.
La fluorescence obtenue après six heures est lue en plaque 96-puits dans un fluorimètre équipé avec une combinaison de filtres de 360 nm et 460 nm pour l'excitation et l'émission. Les composés de l'invention ont des IC₅₀ pour l'ensemble des MMPs à l'exception de la MMP-1, comprises entre 10⁻¹⁰ et 10⁻⁸M. Une spécificité d'un facteur 1000 existe pour les collagénases MMP-13 et MMP-8 vis-à-vis de la collagénase MMP-1.

### EXEMPLE B : Dégradation de la matrice du cartilage in vitro

Les composés de l'invention ont été étudiés sur un modèle de destruction de la matrice du cartilage induite par l'IL-1β. Les essais, réalisés sur cartilage de lapin, concernent :
- d'une part, la dégradation du collagène : dosage colorimétrique selon la technique de Grant (GRANT R.A. Estimation of OH-proline by the autoanalyser, J. Clin. Path., 17, 685, 1964) de la fraction d'OH-proline relarguée par le tissu mis en contact pendant 2 jours avec l'IL-1β (10 ng/ml) et de la plasmine (0.1 U/ml) ;
- d'autre part, la dégradation des protéoglycanes : mesure radio-isotopique de la fraction de glycosaminoglycanes relargués par le tissu préalablement marqué au ³⁵SO₄, au cours des 24 heures de contact avec l'APMA (5x10⁻⁴M), faisant suite à 24 heures de stimulation par l'IL-1β (10 ng/ml).

Les composés de l'invention ont été étudiés par adjonction au milieu de culture pendant les 3 jours de l'essai. Pour des concentrations comprises entre 10⁻⁸ et 10⁻⁶M, ils se sont fortement opposés à la dégradation du collagène et des protéoglycanes. A titre d'exemple, les activités exercées par certains composés de l'invention sont les suivantes :

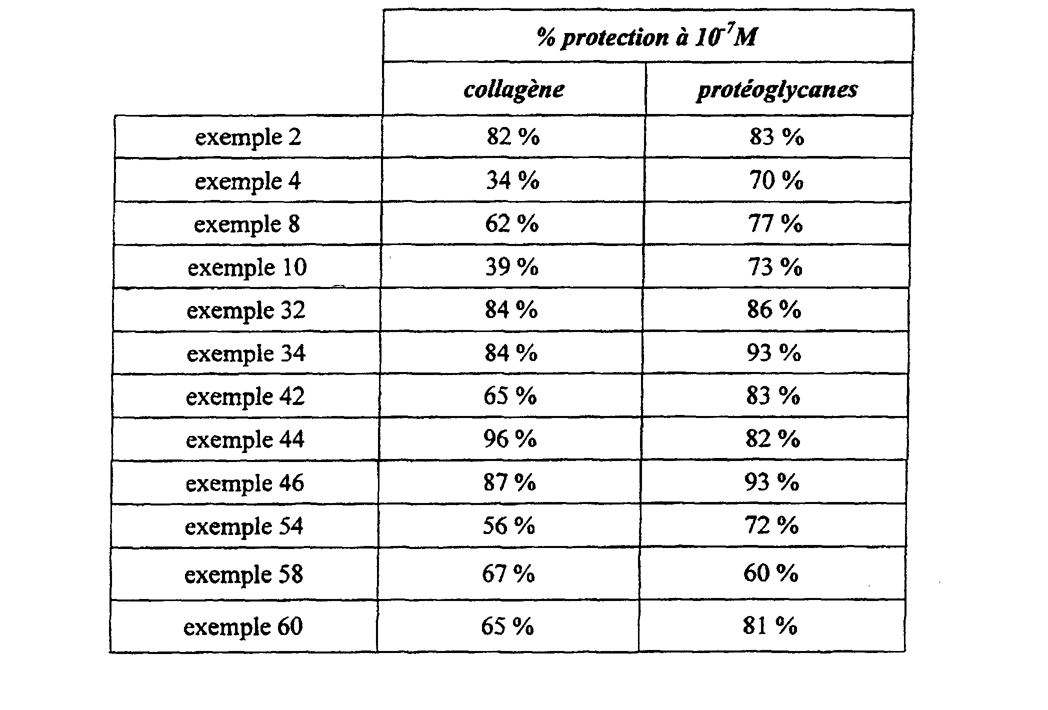

### EXEMPLE C : Angiogénèse in vitro

Des tronçons d'aorte thoracique de rats mâles Fischer 344 âgées de 8 à 12 semaines sont immergés dans un gel de collagène de type I selon la méthode de Nicosia et Ottinetti (1990). Après cinq jours de culture en milieu sans sérum, les préparations sont examinées au microscope et la formation de pseudo-vaisseaux quantifiée en terme de densité vasculaire après digitalisation et analyse d'image.
A titre d'exemple, l'IC₅₀ du composé de l'exemple 2 est égale à 2,3 nM, celle du composé de l'exemple 4 est égale à 100 nM.

### EXEMPLE D : Absorption après traitement par voie orale chez la souris - Bioactivité plasmatique

L'absorption des composés a été évaluée au niveau circulant après traitement oral chez la souris (CD1, mâle, 25-30 g) par mesure du potentiel du plasma à inhiber la MMP-13 selon des conditions expérimentales identiques à celles utilisées *in vitro* (Exemple A). A différents temps suivant l'administration des composés, cette bioactivité a été déterminée sur plasma après élimination des protéines par l'alcool éthylique (18 h à -20°C). A titre d'exemple, les % d'inhibition de la MMP-13 obtenus après 30 mg/kg sont les suivants :

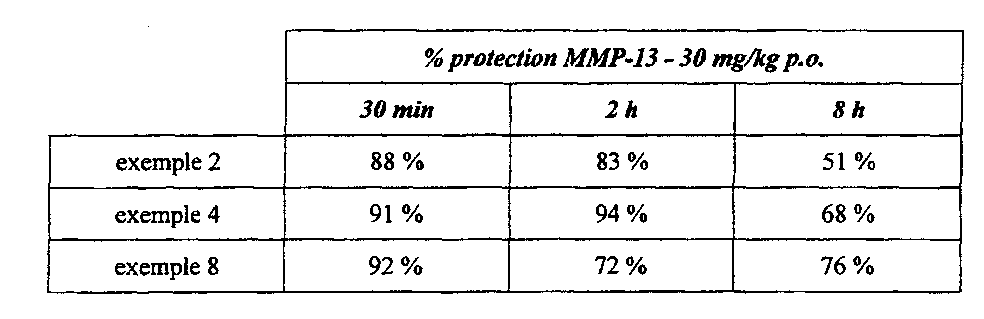

### EXEMPLE E : Arthrite à l'adjuvant de Freund chez le rat - Protection de la dégradation articulaire

L'activité protectrice des composés sur la dégradation du tissu articulaire a été étudiée dans le modèle d'arthrite à l'adjuvant de Freund chez le rat (Lewis, femelle, 62 jours). La pathologie autoimmune induite par injection intraplantaire de 0,1 ml d'adjuvant (Mycobacterium butyricum 4 mg/ml) dans l'une des pattes postérieures, entraîne, en plus d'une réaction inflammatoire, une destruction articulaire. Celle-ci a été évaluée après 21 jours sur la patte non injectée, à la fois au niveau osseux (mesure densitométrique de la partie proximale du fémur) et au niveau du cartilage rotulien (contenus en OH-proline et en glycosaminoglycanes respectivement mesurés selon la technique de Farndale and coll. (Biophysica Acta, 1986, 883, 173-177) et celle décrite par Grant (J. Clin. Path.. 1964, 17, 685)). A titre d'exemple, le composé de l'exemple 2 administré par voie orale 2 fois par jour à la dose de 40 mg/kg (10 animaux/lot) a diminué de 42 % (P < 0,01)la perte en contenu minéral osseux du fémur proximal observée chez les témoins arthritiques et de 61 % (P < 0,05) et 98 % (P < 0,01) la perte respective en glycosaminoglycanes et en OH-proline du cartilage rotulien.

### EXEMPLE F : Composition pharmaceutique

| Formule de préparation pour 1000 comprimés dosés à 100 mg | |
|---|---|
| Composé de l'exemple 2 | 100 mg |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 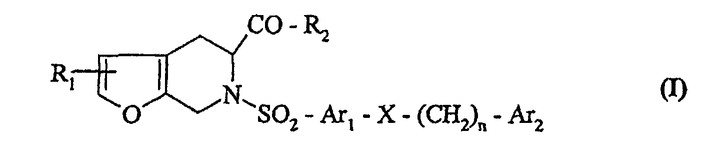 dans laquelle :
R₁ représente un atome d'hydrogène, d'halogène, un groupement alkyle (C₁-C₆) linéaire ou ramifié, ou alkoxy (C₁-C₆) linéaire ou ramifié,
R₂ représente un groupement hydroxy, alkoxy (C₁-C₆) linéaire ou ramifié, ou -NHOH,
Ar₁ représente un groupement phénylène ou biphénylène,
X représente un atome d'oxygène ou de soufre,
R représente un atome d'hydrogène ou un groupement alkyle (C₁-C₆) linéaire ou ramifié,
n représente un nombre entier compris entre 0 et 6 inclus,
Ar₂ représente :
- un groupement phényle substitué par un groupement hétéroaryle,
- un groupement biphényle substitué par un groupement hétéroaryle,
- un groupement pyridinyle, substitué par un groupement hétéroaryle,
- ou un groupement hétérocyclique,
leurs isomères ainsi que leurs sels d'addition à un acide ou à une base pharmaceutiquement acceptable,
étant entendu que :
- par groupement hétéroaryle, on comprend groupement aromatique monocyclique ou un groupement bicyclique dans lequel au moins un des cycles est aromatique, contenant un, deux ou trois hétéroatomes, identiques ou différents, choisis parmi azote, oxygène ou soufre, éventuellement substitué par un ou plusieurs groupements, identiques ou différents, choisis parmi halogène, alkyle (C₁-C₆) linéaire ou ramifié, alkoxy (C₁-C₆) linéaire ou ramifié, trihalogénoalkyle (C₁-C₆) linéaire ou ramifié, trihalogénoalkoxy (C₁-C₆) linéaire ou ramifié, ou hydroxy,
- par groupement hétérocyclique, on comprend un groupement saturé ou partiellement saturé, non aromatique mono ou bicyclique contenant un, deux ou trois hétéroatomes, identiques ou différents, choisis parmi azote, oxygène ou soufre, éventuellement substitué par un ou plusieurs groupements, identiques ou différents, choisis parmi halogène, alkyle (C₁-C₆) linéaire ou ramifié, trihalogénoalkyle (C₁-C₆) linéaire ou ramifié, alkoxy (C₁-C₆) linéaire ou ramifié, trihalogénoalkoxy (C₁-C₆) linéaire ou ramifié, ou hydroxy.

2. Composés de formule (I) selon la revendication 1 **caractérisés en ce que** R₂ représente un groupement -NHOH.

3. Composés de formule (I) selon l'une quelconque des revendications 1, ou 2 **caractérisés en ce que** Ar₁ représente un groupement phénylène et n représente zéro.

4. Composés de formule (I) selon la revendication 3 **caractérisés en ce que** Ar₂ représente un groupement phényle substitué par un groupement hétéroaryle.

5. Composés de formule (I) selon la revendication 4 **caractérisés en ce que** Ar₂ représente un groupement phényle substitué par un groupement choisi parmi imidazolyle, triazolyle ou pyridinyle.

6. Composés de formule (I) selon l'une quelconque des revendications 1 à 5 **caractérisé en ce que** X représente un atome d'oxygène ou de soufre, R₂ représente un groupement -NHOH, Ar₁ représente un groupement phénylène, n représente zéro, Ar₂ représente un groupement phényle substitué par un groupement choisi parmi imidazolyle, triazolyle ou pyridinyle.

7. Composés de formule (I) selon l'une quelconque des revendications 1, ou 2 **caractérisés en ce que** Ar₁ représente un groupement biphénylène et Ar₂ représente un groupement hétérocyclique.

8. Composés de formule (I) selon la revendication 1 qui est le 6-{4-[4-(imidazol-1-yl)phénoxy]benzènesulfonyl}-4,5,6,7-tétrahydrofuro[2,3-c]pyridine-(5R)-(N-hydroxy) carboxamide, ainsi que ses sels d'addition à un acide ou à une base pharmaceutiquement acceptable.

9. Composés de formule (I) selon la revendication 1 qui est le 6-{4'-[2-(pyrrolidin-1-yl)éthoxy]biphényl-4-sulfonyl}-4,5,6,7-tétrahydrofuro[2,3-c]pyridine-(5R)-(N-hydroxy) carboxamide, chlorhydrate, ainsi que ses sels d'addition à un acide ou à une base pharmaceutiquement acceptable.

10. Composés de formule (I) selon la revendication 1 qui est le 6-{4-[4-(1,3,4-triazol-1-yl) phénoxy]benzènesulfonyl}-4,5,6,7-tétrahydrofuro[2,3-c]pyridin-(5R)-(N-hydroxy) carboxamide, ainsi que ses sels d'addition à un acide ou à une base pharmaceutiquement acceptable.

11. Composés de formule (I) selon la revendication 1 qui est le 6-{4-[4-(pyridin-4-yl) phénoxy]benzènesulfonyl}-4,5,6,7-tétrahydrofuro[2,3-c]pyridin-(5R)-(N-hydroxy) carboxamide, ainsi que ses sels d'addition à un acide ou à une base pharmaceutiquement acceptable.

12. Composés de formule (I) selon la revendication 1 qui est le 6-{4-[(4-(1,3,4-triazol-1-yl)phénythio]benzènesulfonyl}-4,5,6,7-tétrahydrofuro[2,3-c]pyridine-(5R)-(N-hydroxy) carboxamide, ainsi que ses sels d'addition à un acide ou à une base pharmaceutiquement acceptable.

13. Procédé de préparation des composés de formule (I) selon la revendication 1 **caractérisé en ce que** l'on utilise comme produit de départ un composé de formule (II), sous forme racémique ou d'isomère défini : 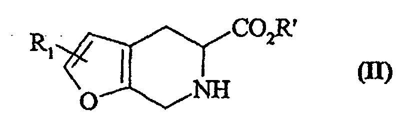 dans laquelle R₁ a la même signification que dans la formule (I), et R' représente un atome d'hydrogène ou un groupement alkyle (C₁-C₆) linéaire ou ramifié, dont on substitue la fonction aminé par un dérivé halogéné de formule (III) :
ClSO₂ - Ar₁ - X - (CH₂)ₙ - Ar₂ (III)
dans laquelle Ar₁, X, n et Ar₂ ont la même signification que dans la formule (I), pour conduire :
- lorsque R' représente un atome d'hydrogène au composé de formule (I/a), cas particulier des composés de formule (I) : 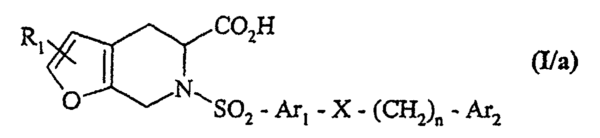 dans laquelle R₁, Ar₁, X, N et Ar₂ sont tels que définis précédemment,
- ou, lorsque R' représente un groupement alkyle (C₁-C₆) linéaire ou ramifié (R") au composé de formule (I/a₁), cas particulier des composés de formule (I) : 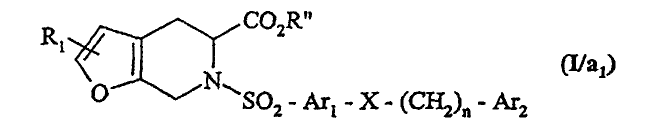 dans laquelle R₁, Ar₁, X, n, Ar₂ et R" sont tels que définis précédemment,
qui peut subir l'action d'un acide, pour conduire au composé de formule (I/a) décrit précédemment,
* composé de formule (I/a) :
que l'on soumet, si on le souhaite, alors à l'action d'une hydroxylamine -O- substituée, pour conduire, après déprotection de la fonction hydroxylamine au composé de formule (I/b), 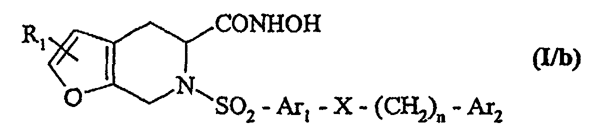 dans laquelle R₁, Ar₁, X, n et Ar₂ sont tels que définis précédemment,
* composés de formule (I/a), (I/a₁) ou (I/b) qui forment l'ensemble des composés de formule (I),
que l'on purifie le cas échéant selon une technique classique de purification, dont on sépare éventuellement les isomères selon une technique classique de séparation et dont on transforme, si on le souhaite, en ses sels d'addition à un acide ou à une base pharmaceutiquement acceptable.

14. Compositions pharmaceutiques contenant comme principe actif un composé selon l'une quelconque des revendications 1 à 12, en combinaison avec un ou plusieurs excipients ou véhicules inertes, non toxiques, pharmaceutiquement acceptables.

15. Compositions pharmaceutiques selon la revendication 14 contenant comme principe actif un composé selon l'une quelconque des revendications 1 à 12 utiles comme inhibiteurs de métalloprotéases.

## Claims

1. Compounds of formula (I): 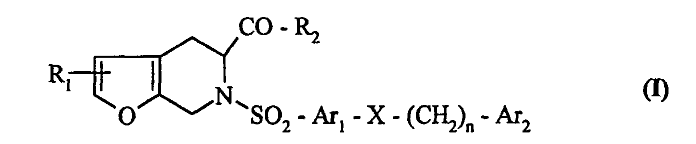 wherein:
R₁ represents a hydrogen or halogen atom, or a linear or branched (C₁-C₆)alkyl or linear or branched (C₁-C₆)alkoxy group,
R₂ represents a hydroxy, linear or branched (C₁-C₆)alkoxy or -NHOH group,
Ar₁ represents a phenylene or biphenylene group,
X represents an oxygen or sulphur atom,
R represents a hydrogen atom or a linear or branched (C₁-C₆)alkyl group,
n is an integer from 0 to 6 inclusive,
Ar₂ represents:
- a phenyl group substituted by a heteroaryl group,
- a biphenyl group substituted by a heteroaryl group,
- a pyridinyl group substituted by a heteroaryl group, or
- a heterocyclic group,
their isomers and addition salts thereof with a pharmaceutically acceptable acid or base, it being understood that:
- "heteroaryl group" is understood to mean a monocyclic aromatic group or a bicyclic group wherein at least one of the rings is aromatic, containing one, two or three identical or different hetero atoms selected from nitrogen, oxygen and sulphur, optionally substituted by one or more identical or different groups selected from halogen, linear or branched (C₁-C₆)alkyl, linear or branched (C₁-C₆)alkoxy, linear or branched trihalo-(C₁-C₆)alkyl, linear or branched trihalo-(C₁-C₆)alkoxy, and hydroxy,
- "heterocyclic group" is understood to mean a saturated or partially saturated, mono- or bicyclic non-aromatic group containing one, two or three identical or different hetero atoms selected from nitrogen, oxygen and sulphur, optionally substituted by one or more identical or different groups selected from halogen, linear or branched (C₁-C₆)alkyl, linear or branched trihalo-(C₁-C₆)alkyl, linear or branched (C₁-C₆)alkoxy, linear or branched trihalo-(C₁-C₆)alkoxy, and hydroxy.

2. Compounds of formula (I) according to claim 1, **characterised in that** R₂ represents an -NHOH group.

3. Compounds of formula (I) according to either claim 1 or claim 2, **characterised in that** Ar₁ represents a phenylene group and n is zero.

4. Compounds of formula (I) according to claim 3, **characterised in that** Ar₂ represents a phenyl group substituted by a heteroaryl group.

5. Compounds of formula (I) according to claim 4, **characterised in that** Ar₂ represents a phenyl group substituted by a group selected from imidazolyl, triazolyl and pyridinyl.

6. Compounds of formula (I) according to any one of claims I to 5, **characterised in that** X represents an oxygen or sulphur atom, R₂ represents an -NHOH group, Ar₁ represents a phenylene group, n is zero, and Ar₂ represents a phenyl group substituted by a group selected from imidazolyl, triazolyl and pyridinyl.

7. Compounds of formula (I) according to either claim 1 or claim 2, **characterised in that** Ar₁ represents a biphenylene group and Ar₂ represents a heterocyclic group.

8. Compound of formula (I) according to claim 1 which is 6-{4-[4-(imidazol-1-yl)phenoxy]benzenesulphonyl}-4,5,6,7-tetrahydrofuro[2,3-*c*]pyridine-(5*R*)-(N-hydroxy)-carboxamide, and its addition salts with a pharmaceutically acceptable acid or base.

9. Compound of formula (I) according to claim 1 which is 6-{4'-[2-(pyrrolidin-1-yl)ethoxy]biphenyl-4-sulphonyl}-4,5,6,7-tetrahydrofuro[2,3-*c*]pyridine-(5*R*)-(N-hydroxy)-carboxamide hydrochloride, and its addition salts with a pharmaceutically acceptable acid or base.

10. Compound of formula (I) according to claim 1 which is 6-{4-[4-(1,3,4-triazol-1-yl)-phenoxy]benzenesulphonyl}-4,5,6,7-tetrahydrofuro[2,3-*c*]pyridine-(5*R*)-(N-hydroxy)-carboxamide, and its addition salts with a pharmaceutically acceptable acid or base.

11. Compound of formula (I) according to claim 1 which is 6-{4-[4-(pyridin-4-yl)-phenoxy]benzenesulphonyl}-4,5,6,7-tetrahydrofuro[2,3-*c*]pyridine-(5*R*)-(N-hydroxy)-carboxamide, and its addition salts with a pharmaceutically acceptable acid or base.

12. Compound of formula (I) according to claim 1 which is 6-{4-[(4-(1,3,4-triazol-1-yl)phenylthio]benzenesulphonyl}-4,5,6,7-tetrahydrofuro[2,3-*c*]pyridine-(5*R*)-(N-hydroxy)-carboxamide, and its addition salts with a pharmaceutically acceptable acid or base.

13. Process for the preparation of compounds of formula (I) according to claim 1, **characterised in that** there is used as starting material a compound of formula (II), in racemic form or in the form of a specific isomer: 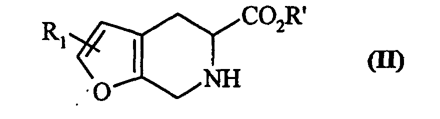 wherein R₁ is as defined for formula (I), and R' represents a hydrogen atom or a linear or branched (C₁-C₆)alkyl group, the amine function of which is substituted by a halogen compound of formula (III):
ClSO₂-Ar₁-X-(CH₂)ₙ-Ar₂ (III)
wherein Ar₁, X, n and Ar₂ are as defined for formula (I),
to yield:
- when R' represents a hydrogen atom, a compound of formula (I/a), a particular case of the compounds of formula (I) : 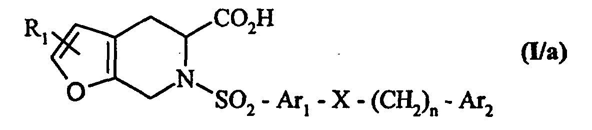 wherein R₁, Ar₁, X, N and Ar₂ are as defined hereinbefore,
- or, when R' represents a linear or branched (C₁-C₆)alkyl group (R"), a compound of formula (I/a₁), a particular case of the compounds of formula (I) : 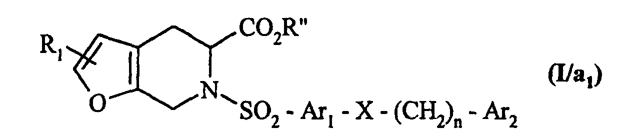 wherein R₁, Ar₁, X, n, Ar₂ and R" are as defined hereinbefore,
which may be subjected to the action of an acid, to yield a compound of formula (I/a) described hereinbefore,
* which compound of formula (I/a) :
is subjected, if desired, to the action of an O-substituted hydroxylamine, to yield, after deprotection of the hydroxylamine function, a compound of formula (I/b), 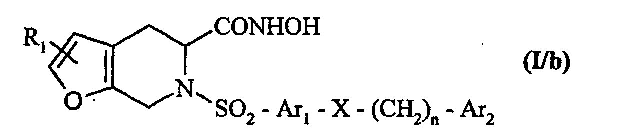 wherein R₁, Ar₁, X, n and Ar₂ are as defined hereinbefore,
* which compounds of formulae (I/a), (I/a₁) and (I/b) constitute the totality of the compounds of formula (I),
which are purified, if necessary, according to a conventional purification technique, are seperated, where appropriate, into their isomers according to a conventional separation technique, and are converted, if desired, into addition salts thereof with a pharmaceutically acceptable acid or base.

14. Pharmaceutical compositions comprising as active ingredient a compound according to any one of claims 1 to 12, in combination with one or more inert, non-toxic pharmaceutically acceptable excipients or carriers.

15. Pharmaceutical compositions according to claim 14 comprising as active ingredient a compound according to any one of claims 1 to 12 for use as metalloprotease inhibitors.

## Patentansprüche

1. Verbindungen der Formel (I): 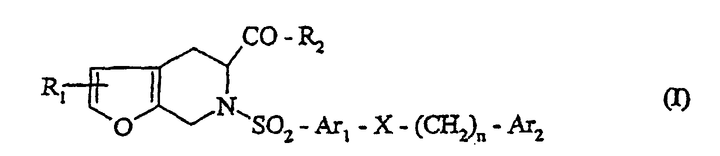 in der:
R₁ ein Wasserstoffatom, ein Halogenatom, eine geradkettige oder verzweigte (C₁-C₆)-Alkylgruppe oder eine geradkettige oder verzweigte (C₁-C₆)-Alkoxygruppe,
R₂ eine Hydroxygruppe, eine geradkettige oder verzweigte (C₁-C₆)-Alkoxygruppe oder eine Gruppe -NHOH,
Ar₁ eine Phenylen- oder Biphenylengruppe,
X ein Sauerstoffatom oder ein Schwefelatom,
R ein Wasserstoffatom oder eine geradkettige oder verzweigte (C₁-C₆)-Alkylgruppe,
n eine ganze Zahl mit einem Wert zwischen 0 und 6 einschließlich und
Ar₂:
- eine Phenylgruppe, die durch eine Heteroarylgruppe substituiert ist,
- eine Biphenylgruppe, die durch eine Heteroarylgruppe substituiert ist,
- eine Pyridinylgruppe, die durch eine Heteroarylgruppe substituiert ist, oder
- eine heterocyclische Gruppe
bedeuten,
deren Isomere sowie deren Additionssalze mit einer pharmazeutisch annehmbaren Säure oder Base,
mit der Maßgabe, daß:
- unter einer Heteroarylgruppe eine monocyclische oder bicyclische aromatische Gruppe zu verstehen ist, bei der mindestens einer der Ringe aromatisch ist, eins, zwei oder drei gleichartige oder verschiedene Heteroatome ausgewählt aus Stickstoff, Sauerstoff oder Schwefel enthält und gegebenenfalls durch eine oder mehrere gleichartige oder verschiedenartige Gruppen ausgewählt aus Halogen, geradkettigem oder verzweigtem (C₁-C₆)-Alkyl, geradkettigem oder verzweigtem (C₁-C₆)-Alkoxy, geradkettigem oder verzweigtem (C₁-C₆)-Trihalogenalkyl, geradkettigem oder verzweigtem (C₁-C₆)-Trihalogenalkoxy oder Hydroxy substituiert ist, zu verstehen ist,
- unter einer heterocyclischen Gruppe eine gesättigte oder teilweise gesättigte, nichtaromatische, mono- oder bicyclische Gruppe zu verstehen ist, die ein, zwei oder drei gleichartige oder verschiedenartige Heteroatome ausgewählt aus Stickstoff, Sauerstoff oder Schwefel enthält und gegebenenfalls durch eine oder mehrere gleichartige oder verschiedene Gruppen ausgewählt aus Halogen, geradkettigem oder verzweigtem (C₁-C₆)-Alkyl, geradkettigem oder verzweigtem (C₁-C₆)-Trihalogenalkyl, geradkettigem oder verzweigtem (C₁-C₆)-Alkoxy, geradkettigem oder verzweigtem (C₁-C₆)-Trihalogenalkoxy oder Hydroxy substituiert ist.

2. Verbindungen der Formel (I) nach Anspruch 1, **dadurch gekennzeichnet, daß** R₂ eine Gruppe -NHOH bedeutet.

3. Verbindungen der Formel (I) nach irgendeinem der Ansprüche 1 oder 2, **dadurch gekennzeichnet, daß** Ar₁ eine Phenylengruppe und n Null bedeuten.

4. Verbindungen der Formel (I) nach Anspruch 3, **dadurch gekennzeichnet, daß** Ar₂ eine durch eine Heteroarylgruppe substituierte Phenylgruppe bedeutet.

5. Verbindungen der Formel (I) nach Anspruch 4, **dadurch gekennzeichnet, daß** Ar₂ eine Phenylgruppe, die durch eine Gruppe ausgewählt aus Imidazolyl, Triazolyl oder Pyridinyl substituiert ist, bedeutet.

6. Verbindungen der Formel (I) nach irgendeinem der Ansprüche 1 bis 5, **dadurch gekennzeichnet, daß** X ein Sauerstoff- oder Schwefelatom, R₂ eine Gruppe -NHOH, Ar₁ eine Phenylengruppe, n Null und Ar₂ eine Phenylgruppe, die durch eine Gruppe ausgewählt aus Imidazolyl, Triazolyl oder Pyridinyl substituiert ist, bedeuten.

7. Verbindungen der Formel (I) nach irgendeinem der Ansprüche 1 oder 2, **dadurch gekennzeichnet, daß** Ar₁ eine Biphenylengruppe und Ar₂ eine heterocyclische Gruppe bedeuten.

8. Verbindungen der Formel (I) nach Anspruch 1, nämlich 6-{4-[4-(Imidazol-1-yl)-phenoxy]-benzolsulfonyl}-4,5,6,7-tetrahydrofuro[2,3-c]pyridin-(5R)-(N-hydroxy)-carboxamid sowie dessen Additionssalze mit einer pharmazeutisch annehmbaren Säure oder Base.

9. Verbindungen der Formel (I) nach Anspruch 1, nämlich 6-{4'-[2-(Pyrrolidin-1-yl)-ethoxy]-biphenyl-4-sulfonyl}-4,5,6,7-tetrahydrofuro[2,3-c]pyridin-(5R)-(N-hydroxy)-carboxamid-Hydrochlorid sowie dessen Additionssalze mit einer pharmazeutisch annehmbaren Säure oder Base.

10. Verbindungen der Formel (I) nach Anspruch 1, nämlich 6-{4-[4-(1,3,4-Triazol-1-yl)-phenoxy]-benzolsulfonyl}-4,5,6,7-tetrahydrofuro[2,3-c]pyridin-(5R)-(N-hydroxy)-carboxamid sowie dessen Additionssalze mit einer pharmazeutisch annehmbaren Säure oder Base.

11. Verbindungen der Formel (I) nach Anspruch 1, nämlich 6-{4-[4-(Pyirin-4-yl)-phenoxyl-benzolsulfonyl}-4,5,6,7-tetrahydrofuro[2,3-c]pyridin-(5R)-(N-hydroxy)-carboxamid sowie dessen Additionssalze mit einer pharmazeutisch annehmbaren Säure oder Base.

12. Verbindungen der Formel (I) nach Anspruch 1, nämlich 6-{4-[(4-(1,3,4-Triazol-1-yl)-phenylthio]-benzolsulfonyl}-4,5,6,7-tetrahydrofuro[2,3-c]pyridin-(5R)-(Nhydroxy)-carboxamid sowie dessen Additionssalze mit einer pharmazeutisch annehmbaren Säure oder Base.

13. Verfahren zur Herstellung der Verbindungen der Formel (I) nach Anspruch 1, **dadurch gekennzeichnet, daß** man als Ausgangsprodukt eine Verbindung der Formel (II) in racemischer Form oder in definierter isomerer Form verwendet: 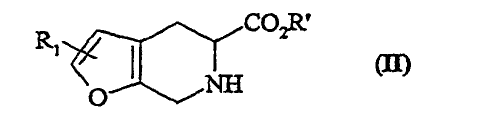 in der R₁ die bezüglich der Formel (I) angegebenen Bedeutungen besitzt und R' ein Wasserstoffatom oder eine geradkettige oder verzweigte (C₁-C₆)-Alkylgruppe darstellt, deren Aminfunktion man mit einem Halogenderivat der Formel (III) substituiert:
ClSO₂ - Ar₁ - X - (CH₂)ₙ - Ar₂ (III)
in der Ar₁, X, n und Ar₂ die bezüglich der Formel (I) angegebenen Bedeutungen besitzen.
zur Bildung:
- dann, wenn R' ein Wasserstoffatom darstellt, der Verbindung der Formel (I/a), einem Sonderfall der Verbindungen der Formel (I): 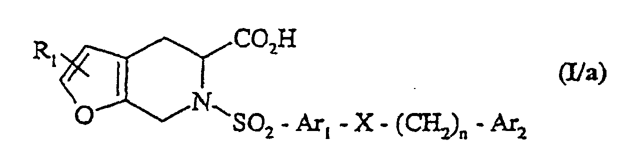 in der R₁, Ar₁, X, n und Ar₂ die oben angegebenen Bedeutungen besitzen,
- oder, wenn R' eine geradkettige oder verzweigte (C₁-C₆)-Alkylgruppe (R") darstellt, der Verbindung der Formel (I/a₁), einem Sonderfall der Verbindungen der Formel (I): 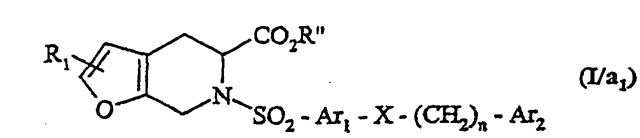 in der R₁, Ar₁, X, n, Ar₂ und R" die oben angegebenen Bedeutungen besitzen, welche man der Einwirkung einer Säure unterwerfen kann zur Bildung der Verbindung der Formel (I/a), wie sie oben definiert worden ist,
* welche Verbindung der Formel (I/a):
man gewünschtenfalls der Einwirkung eines -O-substituierten Hydroxylamins unterwirft, so daß man nach der Abspaltung der Schutzgruppe der Hydroxylaminfunktion zu der Verbindung der Formel (I/b) gelangt: 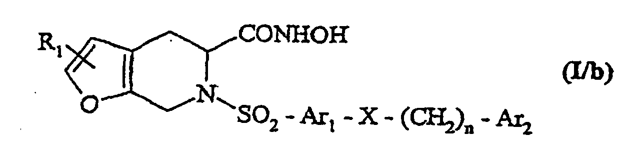 in der R₁, Ar₁, X, n und Ar₂ die oben angegebenen Bedeutungen besitzen,
* welche Verbindungen der Formeln (I/a), (I/a₁) und (I/b), welche die Gesamtheit der Verbindungen der Formel (I) bilden,
man gegebenenfalls mit Hilfe einer klassischen Reinigungsmethode reinigt, man sie gegebenenfalls mit Hilfe einer klassischen Trennmethode in ihre Isomeren auftrennt und welche man gewünschtenfalls in ihre Additionssalze mit einer pharmazeutisch annehmbaren Säure oder Base überführt.

14. Pharmazeutische Zubereitungen enthaltend als Wirkstoff eine Verbindung nach irgendeinem der Ansprüche 1 bis 12 in Kombination mit einem oder mehreren inerten, nichttoxischen, pharmazeutisch annehmbaren Trägermaterialien oder Bindemitteln.

15. Pharmazeutische Zubereitungen nach Anspruch 14 enthaltend als Wirkstoff eine Verbindung nach irgendeinem der Ansprüche 1 bis 12 geeignet als Inhibitor von Metalloproteasen.
